(19) Europäisches Patentamt
European Patent Office
Office européen des brevets

(11) **EP 2 129 453 B1**

(12) **EUROPÄISCHE PATENTSCHRIFT**

(45) Veröffentlichungstag und Bekanntmachung des Hinweises auf die Patenterteilung:
**23.08.2017 Patentblatt 2017/34**

(21) Anmeldenummer: **08707802.8**

(22) Anmeldetag: **25.02.2008**

(51) Int Cl.:
**B01D 69/08** (2006.01)      **D01D 5/20** (2006.01)
**B01D 63/02** (2006.01)      **B01D 61/24** (2006.01)
**A61M 1/16** (2006.01)

(86) Internationale Anmeldenummer:
**PCT/EP2008/001480**

(87) Internationale Veröffentlichungsnummer:
**WO 2008/104351 (04.09.2008 Gazette 2008/36)**

(54) **DIALYSATOR SOWIE VERFAHREN ZU DESSEN HERSTELLUNG**

DIALYZER AND METHOD OF MANUFACTURING THEREOF

DIALYSEUR ET METHODE DE PRODUCTION DE CELUI-CI

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **26.02.2007 DE 102007009208**

(43) Veröffentlichungstag der Anmeldung:
**09.12.2009 Patentblatt 2009/50**

(73) Patentinhaber: **Fresenius Medical Care Deutschland GmbH**
**61352 Bad Homburg (DE)**

(72) Erfinder:
• **HEILMANN, Klaus**
**66606 St. Wendel (DE)**
• **KELLER, Torsten**
**54411 Hermeskeil (DE)**

(74) Vertreter: **Herrmann, Uwe et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
EP-A1- 1 344 542     EP-A1- 1 433 490
WO-A-03/106004     JP-A- H0 796 152
US-A- 4 380 520     US-A- 5 730 712

• C Ronco ET AL: "Enhancement of convective transport by internal filtration in a modified experimental hemodialyzer: technical note", Kidney international, 1 September 1998 (1998-09-01), page 979, XP055225327, UNITED STATES Retrieved from the Internet: URL:http://www.nature.com/ki/journal/v54/n 3/pdf/4490329a.pdf [retrieved on 2015-11-03]
• Claudio Ronco ET AL: "Effects of a reduced inner diameter of hollow fibers in hemodialyzers", KIDNEY INTERNATIONAL, vol. 58, no. 2, 1 August 2000 (2000-08-01) , pages 809-817, XP055225333, LONDON, GB ISSN: 0085-2538, DOI: 10.1046/j.1523-1755.2000.00230.x

EP 2 129 453 B1

**Beschreibung**

Die vorliegende Erfindung betrifft Dialysator mit einem Filtergehäuse und wenigstens einem in dem Filtergehäuse angeordneten Faserbündel sowie ein Verfahren zur Herstellung eines solchen Dialysators. Bei der Dialyse wird dem Patienten kontinuierlich Blut entnommen und in einem Kreislauf durch ein Filtermodul (Dialysator) geführt und anschließend dem Patienten zurückgeführt. Bei der Durchströmung des Filters werden dem Blut Stoffwechselprodukte, die nicht mehr über die Niere ausgeschieden werden können, entzogen und es besteht die Möglichkeit, je nach Bedarf dem Blut Bestandteile aus der Dialysierflüssigkeit über den Filter zuzufügen. Ein solches Filtermodul weist üblicherweise ein Bündel aus Hohlfasermembranen auf, das in einem Gehäuse eingefaßt ist. In dem Gehäuse befinden sich die Membranfasern in einer länglichen gestreckten mehr oder weniger parallelen Anordnung. Anschlüsse an dem Gehäuse ermöglichen es, dass das Blut von den Schlauchleitungen des Blutkreislaufs im allgemeinen in das Faserinnere, d.h. in den Faserhohlraum geleitet wird, diesen der Länge nach durchströmen und an den Enden der Fasern durch einen Auslass in die weiterführende Schlauchleitung des Blutkreislaufes geführt wird.

Des Weiteren wird dem die Fasern umgebenden Innenraum des Gehäuses die Dialysierflüssigkeit mittels eines zweiten Flüssigkeitskreislaufes zugeführt. Bei der Dialysierflüssigkeit handelt es sich im allgemeinen um eine wäßrige Flüssigkeit, die den Gehäuseinnenraum beispielsweise in Faserrichtung der Länge nach durchströmt. Zur Zufuhr der Dialysierflüssigkeit in den Gehäuseinnenraum und zur Abfuhr der Dialysierflüssigkeit aus dem Gehäuseinnenraum weist das Gehäuse des Filtermoduls entsprechende Anschlüsse auf. Die Membranfasern stehen somit im Kontakt mit zwei Flüssigkeitsströmen, dem Strom der Dialysierflüssigkeit an der äußeren Membranwand und dem Blutstrom an der inneren Membranwand.

Bestandteile beider Flüssigkeiten (Blut und Dialysierflüssigkeit) können entsprechend ihrer Größe die poröse Membranstruktur durchdringen. Hierbei ist die Porengröße der Membran für die Frage entscheidend, welche Stoffe die Membranwand durchdringen können und welche nicht. Die mittlere Porengröße einer Hohlfasermembran kann durch den Herstellprozeß beeinflußt werden. Je nach Behandlungsverfahren werden unterschiedliche Hohlfasermembranen verschiedener Porengrößen benötigt.

Für Dialyseverfahren sind Membranen mit einer relativ kleinen Porengröße im Gebrauch. Bei diesen Verfahren werden vornehmlich Substanzen von geringer bis mittlerer Größe aus dem Blut entfernt. Im allgemeinen ordnet man Moleküle mit einem Molekulargewicht von < 500 g/mol in Blutbehandlungsverfahren zu der Gruppe der kleinen Moleküle und Moleküle mit einem Molekulargewichtsbereich von 500 - 15.000 g/mol werden der Gruppe mit mittelgroßer Molekülgröße zugeordnet. Bei Plasmaphereseverfahren werden dagegen Membranen mit größeren Porendurchmessern benötigt, da hier das gesamte Blutplasma mit auch hochmolekularen großen Eiweißmolekülen von den Zellbeständen des Blutes abgetrennt werden sollen. Zu diesen großen Molekülen zählen Proteine mit einem Molekulargewicht größer als 15.000 g/mol.

Der Stofftransport durch die Membranwand kann nach verschiedenen Prinzipien von der Blutseite zur Dialysatseite oder aber auch umgekehrt von der Dialysatseite zur Blutseite erfolgen. Im wesentlichen relevant sind hierbei die Transportphänomene der Diffusion und der Konvektion. Die treibende Kraft für die Diffusion ist ein Konzentrationsunterschied, d.h. das Bestreben, dass Konzentrationsunterschiede in flüssigen oder gasförmigen Systemen ausgeglichen werden. An diesem Konzentrationsausgleich sind besonders kleine Moleküle beteiligt, da sie vergleichsweise beweglich sind, d.h. eine ausgeprägte Teilchenbewegung ausführen. Große Moleküle führen dagegen nur sehr geringe Teilchenbewegungen aus und werden aufgrund von Diffusion kaum durch die Membranwand transportiert.

Der transmembrane Transport von mittleren und großen Molekülen kann dagegen durch Konvektion erfolgen. Als Konvektion wird im vorliegenden Fall die durch einen transmembranen Druckgradienten (TMP = transmembrane pressure) erzwungene Strömung durch die Membranwand bezeichnet. Eine Druckdifferenz zwischen Dialysatseite und Blutseite wird durch Flußrate und Strömungsrichtung der beiden Flüssigkeitskreisläufe (Blut und Dialysierflüssigkeit) hervorgerufen.

Der transmembrane Filtratfluß, auch als Ultrafiltrationsrate $Q_F$ bezeichnet, und somit der Flüssigkeitsstrom, der zur Dialysatseite oder Blutseite übertritt, ist gemäß folgender Beziehung proportional zum transmembranen Druck TMP:

$$Q_F = UF_{coeff} \cdot TMP$$

Es wird deutlich, dass der Filtratfluß $Q_F$ um so größer ist je größer der Ultrafiltrationskoeffizienten $UF_{coeff}$ bzw. der transmembrane Druck TMP ist. $UF_{coeff}$ ist dabei ein Maß für die auf die Oberfläche bezogene Permeabilität der Membran und er steigt mit der Anzahl der Poren, der Membranoberfläche und der Größe der Poren.

Membranen, die diese optimierten Parameter aufweisen, werden auch als high flux Membranen bezeichnet. Sie werden so bezeichnet, da sie einen hohen transmembranen Fluß ermöglichen und somit auch einen optimierten Stofftransport mittlerer und großer Moleküle ermöglichen.

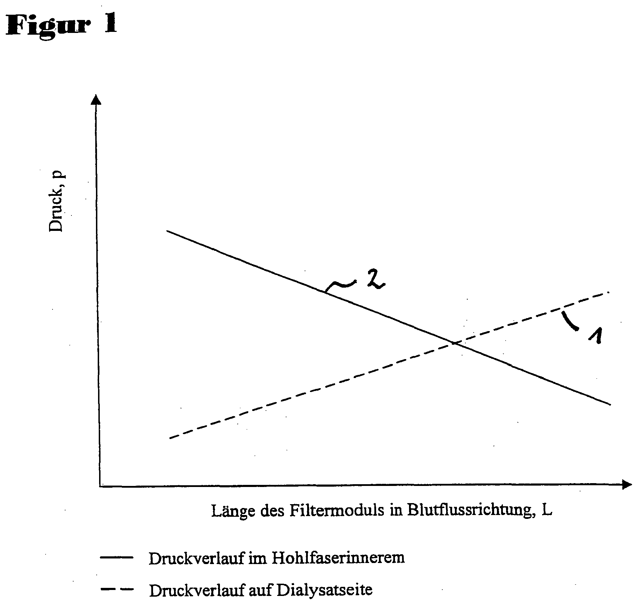

Das Prinzip des konvektiven Transportes wird nun schematisch anhand von Figur 1 erläutert.

Figur 1 zeigt den in einem high flux Filtermodul zu findenden Druckverlauf auf der Dialysatseite (Linie 1) und im Inneren der Hohlfasern (Linie 2) über die Länge der Hohlfasern in Flußrichtung des Blutes.

Beim Bluteintritt in das Filtermodul bzw. in die Hohlfasern liegt zunächst ein hoher Druck auf der Blutseite vor, der kontinuierlich und linear bis zum Blutaustritt abnimmt. Der Druck auf der Dialysatseite zeigt ein entgegengesetztes Verhalten, da in dem hier dargestellten Fall die Strömungsrichtungen umgekehrt sind, d.h. der Filter wird im Gegenstrom betrieben. In einem solchen Filtermodul stellt die Fläche, die zwischen der Geraden des blutseitigen Druckverlaufs und der Geraden des dialysatseitigen Druckverlaufs in einem Längenabschnitt, $L_2$-$L_1$ liegt, ein Maß für den TMP da. Je größer der Abstand beider Geraden ist, desto größer ist an der angegebenen Stelle der transmembrane Druck. In Figur 1 erkennt man, dass die Fläche in solchen gleich bleibenden Längenabschnitten mit zunehmender Länge, d.h. mit zunehmendem Abstand vom Ende oder Anfang des Filters kleiner wird, beim Schnittpunkt der Geraden Null ist und bei größerer Länge wieder zunimmt. Daraus folgt, dass der TMP, betrachtet vom Bluteingang (in Figur 1 links dargestellt), kontinuierlich abnimmt. In dem Bereich, in dem der Druck auf der Blutseite größer ist als der Druck auf der Dialysatseite findet zunächst ein konvektiver Transport von der Blutseite auf die Dialysatseite statt. Nach dem Schnittpunkt der Druckgeraden wächst der TMP wieder an, aber ein konvektiver Stofftransport erfolgt nun von der Dialysat- auf die Blutseite, da der dialysatseitige Druck größer ist als der Druck auf der Blutseite.

Bei derartigen Filtermodulen besteht eine Herausforderung darin, den TMP und damit auch den transmembranen Fluß zu steigern. Eine Möglichkeit besteht darin, in dem Filtermodul auf der Dialysatseite eine Flußbarriere einzubauen. Eine derartige Vorgehensweise ist aus der US 5,730,712 bekannt. Schematisch sind die Druckverläufe in einem solchen Modul in Figur 2 gezeigt.

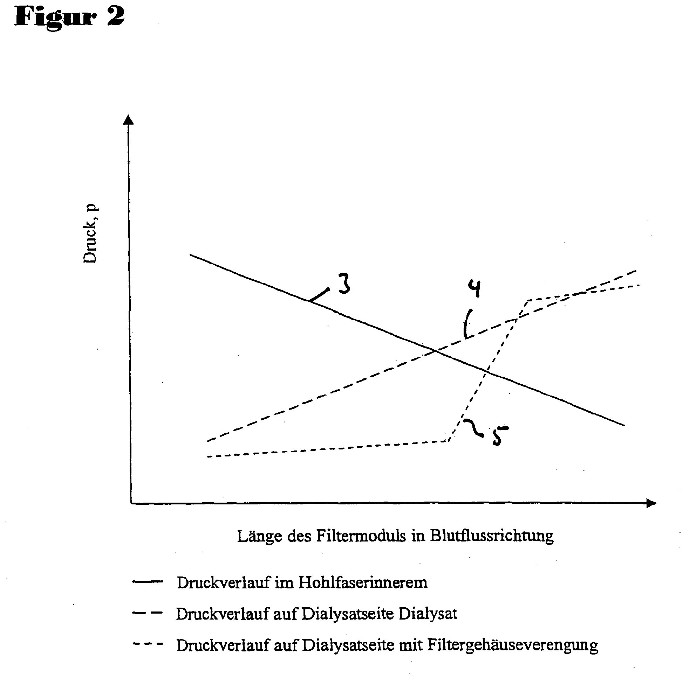

Wie auch in Figur 1 kennzeichnet die durchgezogene Gerade (Linie 3) den Druckverlauf auf der Blutseite, während die gestrichelte Linie 4 den Druckverlauf der der Dialysatseite eines unveränderten Filtermoduls zeigt. Wird nun ein Filter mit einer Flußbarriere auf der Dialysatseite eingesetzt, ergibt sich die gestrichelte Linie 5. Wie dies aus Figur 2 deutlich hervorgeht, bewirkt die Flußbarriere, dass der Druck auf der Dialysatseite, betrachtet in Dialysatflussrichtung (gemäß Figur 2 von rechts nach links), gegenüber dem unmodifizierten Filtermodul zunächst vergleichsweise schwach absinkt, im Bereich der Flußbarriere findet ein starker Druckabfall statt. Stromabwärts der Flußbarriere nimmt der dialysatseitige Druck nur noch wenig ab, d.h. die Steigung des Druckverlaufes über die Länge des Filtermoduls ist geringer als in dem Bereich der dialyseseitigen Flußbarriere.

Aufgrund der Tatsache, dass sich im Bereich der Flußbarriere ein Staudruck aufbaut, muss der Eingangsdruck des einströmenden Dialysats in das Filtergehäuse mit Flußbarriere höher sein als bei einem Filtergehäuse ohne Flußbarriere.

Der Einbau der Flußbarriere führt dazu, dass der Betrag der eingeschlossenen Fläche zwischen blutseitigem Druckverlauf und dialysatseitigem Druckverlauf größer ist als beim Filtermodul, das eine solche Flußbarriere nicht aufweist, wie dies deutlich aus Figur 2 hervorgeht. Es ergibt sich daher in einem solchen Modul ein größerer konvektiver transmembraner Transport als bei einem Filtermodul ohne die genannte Flußbarriere. Der Flußwiderstand auf der Dialysatseite bewirkt somit eine Vergrößerung des transmembranen Flusses von der Blut- zur Dialysatseite. Dieses Prinzip ist von Ronco et al. untersucht worden. Wir nehmen Bezug auf den Artikel C. Ronco et al.: "Enhancement of convective transport by internal filtration in a modified experimental hemodialyzer"; Kidney International, Vol. 54; 1998, S. 979 ff., aus dem sich ergibt, dass die Entfernung von mittelgroßen Molekülen, Vitamin B12 und Insulin aufgrund der Verringerung des Strömungsquerschnittes auf der Dialysatseite deutlich gesteigert werden konnte. Dieses Prinzip ist auch Gegenstand der EP 1 433 490, EP 1 344 542 sowie der WO 2006/024902.

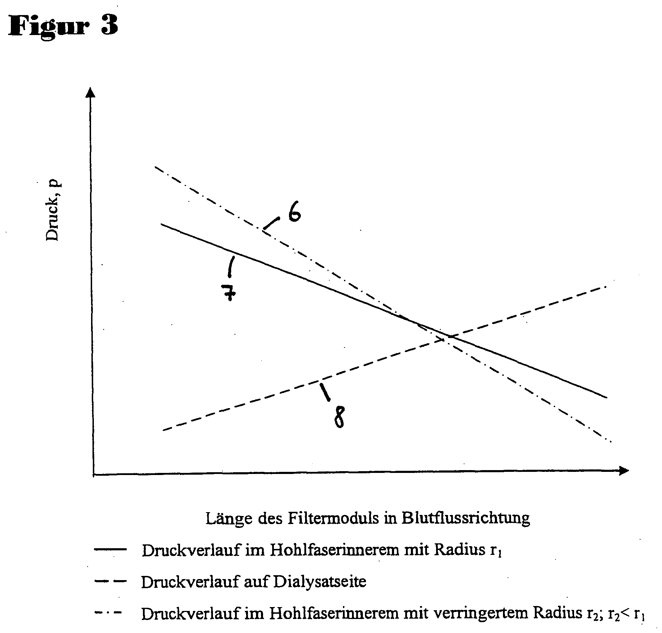

Des weiteren ist es bekannt, den transmembranen Druck dadurch zu steigern, Hohlfasern mit kleinem Innendurchmesser eingesetzt werden. Figur 3 zeigt den Einfluß des Faserinnendurchmessers auf den Faserinnendruck über die Faserlänge. Gemäß des Gesetzes von Hagen-Poiseuille nimmt der Druckabfall dabei mit der Abnahme des Kapillarradius zu. Daraus ergibt sich, dass der Druckverlauf innerhalb einer Hohlfaser (Figur 3, Linie 6) mit kleinerem Durchmesser steiler ausgeprägt ist, als bei Hohlfasern mit demgegenüber größerem Durchmesser (Figur 3, Linie 7). Linie 8 kennzeichnet den Druckverlauf auf der Dialysatseite. Aus Figur 3 ergibt sich, dass sich die Fläche in einem Längenabschnitt, $L_2$-$L_1$ zwischen den jeweiligen Geraden des Druckverlaufes auf der Dialysatseite und auf der Blutseite und somit der transmembrane Druck durch eine Verringerung des Hohlfaserradius vergrößert.

Ronco et al. untersuchte auch dieses Prinzip zur TMP Steigerung. Wir nehmen Bezug auf Ronco et al.: "Effects of a reduced inner diameter of hollow fibers in hemodialyzers"; Kidney International, Vol. 58; 2000, S. 809 ff.. Bei der vergleichenden Untersuchung von Hohlfasermembranen mit 200 $\mu$m und 185 $\mu$m Innendruchmesser, wurde für die Membran mit geringerem Innendruchmesser eine verbesserte Entfernung der mittelgroßen Moleküle Vitamin B12 und Inulin festgestellt.

Die Verringerung des Faserinnendurchmessers führt allerdings zu Problemen. Beim Einleiten des Blutes in die sehr kleinen Faseröffnungen werden die Blutzellen in diesem Strömungsabschnitt stark beschleunigt und es kommt zu großer Reibung zwischen Blutzellen und dem Material des Filtermoduls. Blutzellen können unter diesen Umständen zerstört werden. Der Innenradius lässt sich daher nicht beliebig verringern. Handelsübliche Filtermodule weisen unter anderem Hohlfaser-

radien von 185 μm auf. Kleinere Radien sind in der extrakorporalen Blutbehandlung aber als ungünstig einzustufen.

Die WO 03/106004 A offenbart ein Filtermodul für eine Vielzahl von industriellen Anwendungen. Die einem Dialysator innewohnende Eigenschaft einen Stoff- und Flüssigkeitstransport in beide Richtungen einer semipermeablen Membran zu transferieren, ist in der vorstehend aufgeführten Druckschrift nicht beschrieben.

Die JP H07 96152 A beschreibt ein zur Wasserreinigung verwendbares Filtermodul mit der hierfür typischen "Dead-End"-Filterstruktur, also einer Zuflussöffnung für ein zu reinigendes Medium und einer Abflussöffnung für das gereinigte Medium.

Es ist daher eine Aufgabe der vorliegenden Erfindung, einen Filter bereitzustellen, bei dem der genannte Nachteil einer Beschädigung oder Zerstörung von Blutzellen nicht oder nur in untergeordnetem Ausmaß auftritt und bei dem dennoch ein ausgeprägter konvektiver Transport über die Membran der Hohlfasern realisiert werden kann.

Diese Aufgabe wird durch einen Dialysator mit den Merkmalen des Anspruchs 1 sowie durch ein Verfahren mit den Merkmalen des Anspruchs 13 gelöst.

Gemäß der vorliegenden Erfindung ist es möglich, eine Hohlfasermembran bereitzustellen, die es ermöglicht, in Blutreinigungsmodulen bei schonender Behandlung des Blutes einen vergleichsweise hohen TMP einzustellen und damit verbunden einen verbesserten konvektiven Transport von mittleren und gegebenenfalls auch großen Molekülen zu ermöglichen.

Die Engstelle kann sich beispielsweise über einen Längenabschnitt der Hohlfaser erstrecken, wobei der Längenabschnitt weniger als 5 %, zwischen 5 % und 10 %, zwischen 10 % und 15 %, zwischen 15 % und 25 % oder mehr als 25 % der gesamten Länge der Faser betragen kann.

Denkbar ist auch, dass sich zu beiden Seiten der Engstelle der Innendurchmesser der Hohlfaser bis zu deren Enden vergrößert, wobei die Engstelle in der Mitte der Länge der Hohlfaser oder auch dazu versetzt angeordnet sein kann.

Der Innendurchmesser der Hohlfaser kann in der Engstelle weniger als 40 %, zwischen 40 % und 50 %, zwischen 50 % und 80 % oder mehr als 80 % des Innendurchmessers der Hohlfaser in dem oder den nicht durch die Engstelle gebildeten Abschnitten der Hohlfaser betragen.

Wie oben ausgeführt, kann sich die Engstelle über einen Längenabschnitt der Hohlfaser erstrecken und einen konstanten Innendurchmesser aufweisen. Auch ist es denkbar, dass sich die Engstelle über einen Längenabschnitt der Hohlfaser erstreckt und einen beispielsweise zunächst abnehmenden und sodann wieder zunehmenden Innendurchmesser aufweist.

Der Übergang von dem oder den der Engstelle benachbarten Bereichen zu der Engstelle kann stufig oder auch stetig ausgeführt sein.

Bei Verengungsstellen in der Faser ist man prinzipiell vor das Problem gestellt, dass strömendes Blut in der Engstelle auf eine höhere Flußgeschwindigkeit beschleunigt wird. Hierbei nehmen auch die Reibungskräfte an der Faserwandung zu und es besteht die Gefahr der Hämolyse. Denkbar ist es daher, dass sich die Engstelle über einen Längenabschnitt der Hohlfaser erstreckt und dass ein oder zwei Übergangsbereiche zwischen der Engstelle und nicht durch die Engstelle gebildeten Bereichen der Hohlfaser existieren. Diese Übergangsbereiche können eine Länge aufweisen, die zwischen 5 % und 20 % der Länge des genannten Längenabschnittes beträgt. Liegt an einer Hohlfaser der Übergangsbereich von größerem zu kleinerem Innendurchmesser auf einem nur sehr kurzen Abschnitt vor, so findet man auch eine starke Blutflußbeschleunigung und hohe Reibungswerte vor. Vorteilhaft ist es daher, dass dieser Übergangsbereich gleichförmig über eine gewisse Abschnittslänge erfolgt. Abschnittlängen für den Übergangsbereich liegen vorzugsweise nicht unter 5 % der gesamten Verjüngungspassage. Es sind aber auch Werte von 10 %, 15 % oder 20 %, je nach Gesamtlänge des Verjüngungsabschnitts möglich.

Ferner kann der Durchmesser kontinuierlich über den gesamten Verjüngungsabschnitt zunächst bis zu einem Minimalwert abnehmen und anschließend wieder bis zum ursprünglichen Innendurchmesser zunehmen. Im Allgemeinen ist es für den vorgesehenen Einsatz der erfindungsgemäßen Faser in Blutbehandlungstherapien vorteilhaft, wenn die Verjüngungsstelle in ihrer Geometrie symmetrisch zu der Stelle mit dem kleinsten Innendurchmesser angeordnet ist. Asymmetrische Geometrien sind aber ebenso geeignet.

Der Innendurchmesser der Verjüngung richtet sich nach der Blutbehandlungsmethode, für die die erfindungsgemäße Faser vorgesehen ist. Hohlfasermembranen, die z. B. zur Plasmapherese eingesetzt werden, haben einen Durchmesser im Bereich von 320 μm. Bei solchen Verfahren ist es zweckmäßig eine stärker ausgeprägte Verjüngung von ca. 50 % oder mehr zu wählen. Eine bevorzugte erfindungsgemäße Faser für dieses Verfahren hat an der verjüngten Stelle einen Innendurchmesser von 150 μm.

Bei für die Dialyse bestimmten Hohlfasern liegt hier der herkömmliche Innendurchmesser bei ca. 200 μm. Eine bevorzugte erfindungsgemäße Ausführung einer Hohlfaser für die Dialyse weist hier ebenfalls einen Innendurchmesser von ca. 150 μm auf. Die relative Verjüngung beträgt hier 25 %.

Der absolute Minimalwert des Innendurchmessers ist gewissen Beschränkungen unterworfen. Bei zu geringem Innendurchmesser wird das Blut entlang der Engpassage zu sehr beschleunigt und es besteht die Gefahr, dass durch Reibungen mit der Faserinnenwand Blutzellen zerstört werden und Hämolysereaktionen auftreten. Vorzugsweise liegt der engste Bereich, d.h. der Bereich der ausgeprägtesten Verjüngung, für Fasern, die in extrakorporalen Blutreinigungsverfahren verwendet werden, abhängig von der Blutflußrate, im Bereich von 150 μm. Es können aber bei Anpassung der Blutflußrate oder anderen Parametern der Blutbehandlung auch kleinere Innendurchmesser der Engstelle gewählt werden.

Die Lage der Engstelle zwischen den Enden der Hohlfaser ist je nach Anforderung variierbar. Typische Faserbündellängen, die in Filtermodulen für die Dialyse eingesetzt werden liegen, zwischen 24 und 28 cm. Innerhalb dieser Abmessungen kann die Verjüngungsstelle je nach Behandlungsmethode an verschiedenen Stellen liegen. Bevorzugt befindet sich die Engstelle im mittleren Abschnitt der Faserbündellänge. Es kann aber auch erwünscht sein, dass der konvektive Transport maßgebend nur von der Blut- auf die Dialysatseite erfolgen soll. In solchen Behandlungsanwendungen wird die Verjüngungsstelle des Faserbündels im Filtermodul vorzugsweise im letzten Drittel, betrachtet in Blutflussrichtung, liegen. Im umgekehrten Fall kann es erwünscht sein, dass der konvektive Transport maßgebend von der Dialysat- auf die Blutseite erfolgt. Bei solchen Anwendungen befindet sich die Verjüngungsstelle vorzugsweise im ersten Drittel des Faserbündels im Filtermodul, wieder betrachtet in Blutflußrichtung.

Die Erfindung betrifft des Weiteren ein Filtermodul mit einem Filtergehäuse und wenigstens einem in dem Filtergehäuse angeordneten Faserbündel.

Besonders vorteilhaft für die angestrebte Steigerung des transmembranen Druckes ist es, wenn die erfindungsgemäße Hohlfaser bzw. das erfindungsgemäße Hohlfaserbündel in einem Filtermodul eingesetzt wird, das auf der Dialysatseite eine Flußrestriktion aufweist. Dementsprechend kann vorgesehen sein, dass Flussrestriktionsmittel vorgesehen sind, durch die die Fläche des die Hohlfasern des Faserbündels umgebenden Bereiches innerhalb des Filtergehäuses, d.h. der Strömungsquerschnitt auf der Dialysatseite verringert wird.

Die Flussrestriktionsmittel können als Engstelle des Filtergehäuses ausgeführt sein, in der der Innendurchmesser des Filtergehäuses gegenüber dem oder den Abschnitten des Gehäuses, die an die Engstelle angrenzen, verringert ist.

Auch ist es denkbar, dass die Flussrestriktionsmittel als Ring ausgeführt sind, der innerhalb des Filtergehäuses angeordnet ist und der das Faserbündel umgibt. Auch auf diese Weise ergibt sich eine Verringerung des Strömungsquerschnittes auf der Dialysatseite. Bei den Flußrestriktionsmitteln kann es sich beispielsweise auch um eine quellbare Substanz handeln, die bei Kontakt mit einem Medium, vorzugsweise bei Kontakt mit der Dialysierflüssigkeit, das/die den das Faserbündel umgebenden Raum durchströmt, aufquillt.

Die vorliegende Erfindung betrifft des Weiteren ein Verfahren zur Herstellung einer Hohlfaser, wobei das Verfahren das Aufwickeln der hergestellten Hohlfaser oder des hergestellten Hohlfaserbündels auf eine Aufwickelvorrichtung, insbesondere auf eine Haspel umfaßt.

Ein derartiges Herstellungsverfahren für Hohlfasermembranen ist hinlänglich aus dem Stand der Technik bekannt. Die Herstellung kann beispielsweise durch ein Naßspinnverfahren erfolgen. Hierbei wird eine Polymerlösung über eine Ringdüse extrudiert und in ein Koakulationsbad eingeleitet. Mit der Extrusion der Polymerlösung wird gleichzeitig durch eine innere Öffnung der Düse ein inneres Fällmedium koextrudiert, so dass insgesamt ein hohler Polymerlösungsfaden gefüllt mit einem Fällmedium vorliegt. Durch einen Phaseninversionsprozeß entsteht aus dem Polymerlösungsfaden eine poröse Hohlfasermembran, die über ein Rollensystem abgezogen wird und durch mehrere Spül- und Behandlungs- und Trocknungsphasen geführt wird. Schließlich wird die Faser auf eine Aufwickelvorrichtung, vorzugsweise auf eine Haspel gewickelt. Bezüglich eines derartigen Herstellverfahrens verweisen wir auf die EP 0 750 936 B1, EP 1 547 628 A1 sowie auf die EP 0 543 355 B1.

Um eine Verengung oder eine Variation des Innendurchmessers zu erzeugen, besteht die Möglichkeit während des Extrudierens der Polymerlösung kurzfristig den Extrusionsdruck des inneren Fällmediums abzusenken. Alternativ ist es auch möglich die Abzugsgeschwindigkeit des Rollensystems zu variieren, mit dem der Polymerfaden abgezogen wird. Bei einer kurzfristigen Erhöhung der Abzugsgeschwindigkeit wird der extrudierte Polymerlösungsfaden in die Länge gezogen und verringert dabei zwangsläufig seinen Innendurchmesser. Eine weitere Methode zur Reduzierung des inneren Strömungsquerschnitts einer Hohlfaser besteht darin, die Faser durch Präge- oder Riffelwalzen derart zu verarbeiten, dass durch den äußeren Druck, den diese Walzen auf die Faser ausüben, die Fasern an dieser Stelle zusammengedrückt werden. Dadurch wird der innere Querschnitt verringert.

Aus der DE 28 42 958 A1 ist es bekannt, durch periodische Druckvariationen während des Extrudierens der Spinnmasse oder des inneren Fällmittels eine Variation des Innendurchmessers zu erzielen. Die Faser erhält so eine im Längsschnitt wellenartige Struktur. Ähnliche Verfahren werden auch in der US 4,380,520 und US 4,291,096 beschrieben. Derartig strukturierte Fasern sollten ein gegenseitiges Anhaften der Fasern im Faserbündel während der Dialyseverfahren vermeiden um damit einen transmembranen Stofftransfer zu verbessern. Eine Steigerung des transmembranen Druckes läßt sich bei derartigen Fasern jedoch nicht beobachten.

Erfindungsgemäß ist ein Verfahren nach Anspruch 13 vorgesehen. Das sieht unter Anderem vor, dass eine Engstelle der Hohlfaser erzeugt wird, in der der Innendurchmesser der Hohlfaser gegenüber dem oder den Abschnitten der Hohlfaser, die an die Engstelle angrenzen, verringert ist und dass der Zeitpunkt, in dem die Aufwickelvorrichtung eine besteimmte Position einnimmt, derart mit dem Zeitpunkt der Erzeugung der Engstelle der Hohlfaser synchronisiert wird, dass sich die Engstelle im aufgewickelten Zustand der Hohlfaser an einer vorgegebenen Position relativ zu der Aufwickelvorrichtung befindet. Die Erfindung schlägt vor, die Haspelstellung mit der Variation des Fällmitteldrucks, mit der Variation der Abzugsgeschwindigkeit oder mit dem Wirken der Präge- und Riffelwalzen zu synchronisieren.

Möglich ist es, dass zur Erzeugung eines Synchronisationssignals beispielsweise am Umfang der Haspel ein Näherungsschalter stationär positioniert wird. Der Näherungsschalter liefert ein Signal beim Durchgang eines Haspelarms durch eine vorgegebene Position. Das Signal wird an eine Prozeßsteuerungseinheit geliefert. Darauf folgend wird durch die Prozeßsteuerungseinheit beispielsweise auf Fällmitteldruck, Abzugsgeschwindigkeit oder Prägewalzen Einfluß genommen. In Folge dessen entsteht eine Engstelle im extrudierten Polymerlösungshohlfaden.

In weiterer Ausgestaltung der Erfindung ist vorgesehen, dass es sich bei der Aufwickelvorrichtung um eine Haspel handelt, die wenigstens ein Haspelsegment aufweist, und dass die Synchronisation der Erzeugung der Engstelle mit der Position der Haspel derart vorgenommen wird, dass die Engstellen jeweils mittig in den Haspelsegmenten liegen. Auch ist es denkbar, dass sich die Engstellen jeweils nicht mittig in den Haspelsegmenten, sondern dazu versetzt befinden. Dementsprechend ist es auch möglich, dass die Engstellen nicht mittig oder symmetrisch zu der Haspelsegmentmitte abgelegt werden. Durch Verlängerung oder Verkleinerung der Totzeitspanne, d.h. der Zeitspanne, die die Engstelle für das Durchlaufen der Strecke zwischen der Extrusionsdüse bzw. zwischen der Einheit zur Herstellung der Engstelle und der gewünschten Position auf dem Haspelrad benötigt, ist es möglich die Lage der Engstellen innerhalb eines Haspelsegments zu variieren. Dadurch ist es möglich, Hohlfaserbündel zu isolieren, deren Engstelle nicht mittig im Bündel vorzufinden sind. Die Lage der Engstellen eines Faserbündels in einem Filtermodul kann somit uneingeschränkt vorherbestimmt werden. Wie oben erwähnt, kann es je nach Blutbehandlungsmethode wünschenswert sein, dass die Faserbündelengestelle, betrachtet in Blutflussrichtung im ersten, zweiten oder dritten Drittel des Filtermoduls liegt. In besonderen Fällen ist es auch möglich, kleinere Abschnitte, in denen die Faserbündelengstelle liegen soll zu wählen.

In weiterer Ausgestaltung der Erfindung ist vorgesehen, dass es sich bei der Aufwickelvorrichtung um eine Haspel handelt, die wenigstens ein Haspelsegment aufweist, und dass die Synchronisation des Zeitpunktes der Erzeugung der Engstelle mit dem Zeitpunkt, in dem die Haspel durch eine bestimmte Position läuft, derart vorgenommen wird, dass pro Haspelsegment mehr als eine Engstelle der Hohlfasern vorliegt. Es somit möglich, mittels des erfindungsgemäßen Verfahrens aus einem Haspelsegment nicht nur ein, sondern mehr als ein Faserbündel zu gewinnen. Dies ist vor allem für Haspeln relevant, die eine kleinere Anzahl von Segmenten, z. b. zwei bis sechs Segmente, und größere Radien der Haspelarme besitzen.

Es müssen lediglich zwei oder mehrere Signale nach dem Durchgang eines Haspelsegments und vor dem Durchgang des nächsten Haspelsegments übermittelt werden, damit die Erzeugung der Engstellen mit der Position der Haspelarme synchronisiert werden kann.

Grundsätzlich ist die Anzahl der Haspelsegmente zur Durchführung des erfindungsgemäßen Verfahrens nicht beschränkt. Haspeln mit elf, neun, sieben Segmenten sind ebenso einsetzbar wie Haspeln mit drei oder fünf Segmenten.

Weitere Einzelheiten und Vorteile der Erfindung werden anhand eines in der Zeichnung dargestellten Ausführungsbeispiels näher erläutert. Es zeigen:

Figur 1:     den Druckverlauf in schematischer Darstellung auf der Blutseite und auf der Dialysatseite über die Länge des Filtermoduls in Blutflußrichtung;

Figur 2:     den Druckverlauf in schematischer Darstellung auf der Blutseite und auf der Dialysatseite über die Länge des Filtermoduls in Blutflußrichtung mit und ohne Verringerung des Strömungsquerschnittes auf der Dialysatseite;

Figur 3:     den Druckverlauf in schematischer Darstellung auf der Blutseite und auf der Dialysatseite über die Länge des Filtermoduls in Blutflußrichtung für unterschiedliche Hohlfaserinnendurchmesser;

Figur 4:     den Druckverlauf in schematischer Darstellung auf der Blutseite und auf der Dialysatseite über die Länge des Filtermoduls in Blutflußrichtung mit und ohne Verringerung des Innendurchmessers der Hohlfasern;

Figur 5:     eine schematische Darstellung der Komponenten des Herstellprozesses von Hohlfasern bzw. Hohlfaserbündeln;

Figur 6:     den Druckverlauf in schematischer Darstellung auf der Blutseite und auf der Dialysatseite über die Länge des Filtermoduls in Blutflußrichtung mit Verringerung des Innendurchmessers der Hohlfasern und mit Verringerung des Strömungsquerschnittes auf der Dialysatseite;

Figur 7:     eine schematische Darstellung eines Filters gemäß der Erfindung mit Verringerung des Innendurchmessers der Hohlfasern im Bereich einer Engstelle;

Figur 8:     eine schematische Darstellung eines Filters gemäß der Erfindung mit Verringerung des Innendurchmessers der Hohlfasern im Bereich einer Engstelle sowie mit Verringerung des Innendurchmessers des Filtergehäuses und

Figur 9:    eine schematische Darstellung eines Haspelrades.

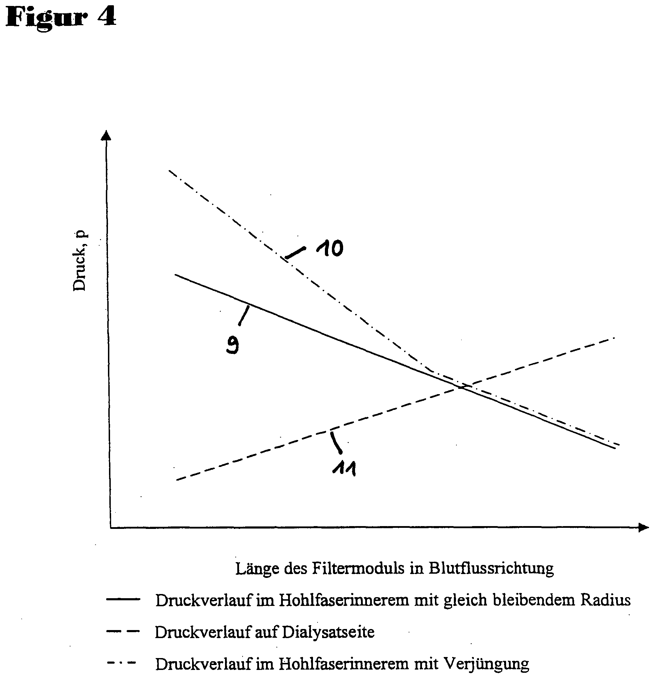

Figur 4 zeigt den Druckverlauf (Linie 9) auf der Blutseite einer Hohlfaser, die auf ihrer gesamten Länge einen gleichbleibenden Innnendurchmesser aufweist, über die Länge des Filtermoduls bzw. der Hohlfaser in Blutflußrichtung. Line 10 in Figur 4 zeigt den Druckverlauf im Inneren einer erfindungsgemäßen Hohlfaser, die eine Engstelle aufweist, in der der Innendurchmesser der Hohlfaser gegenüber der Engstelle benachbarten Bereichen verringert ist. Durch die Engstelle der Hohlfaser liegt an der Fasereintrittsöffnung bei gegebener Flußrate in der in Figur 4 dargestellten Ausführungsform ein höherer Druck vor als bei einer Hohlfaser ohne Engstelle. Wie dies aus dem Kurvenverlauf gemäß Linie 10 hervorgeht, nimmt der Druck entlang der Faser in Flussrichtung bis zur Engstelle relativ gesehen nur langsam ab, fällt im Bereich der Engstelle steil ab und stromabwärts der Engstelle mit einer Steigung, die zwischen der stromaufwärts und der im Bereich der Engstelle liegenden Steigung liegt. Linie 11 zeigt schließlich den Druckverlauf auf der Dialysatseite ebenfalls in Blutflußrichtung, wobei Blut und die Dialysierflüssigkeit den Filter im Gegenstrom durchströmen.

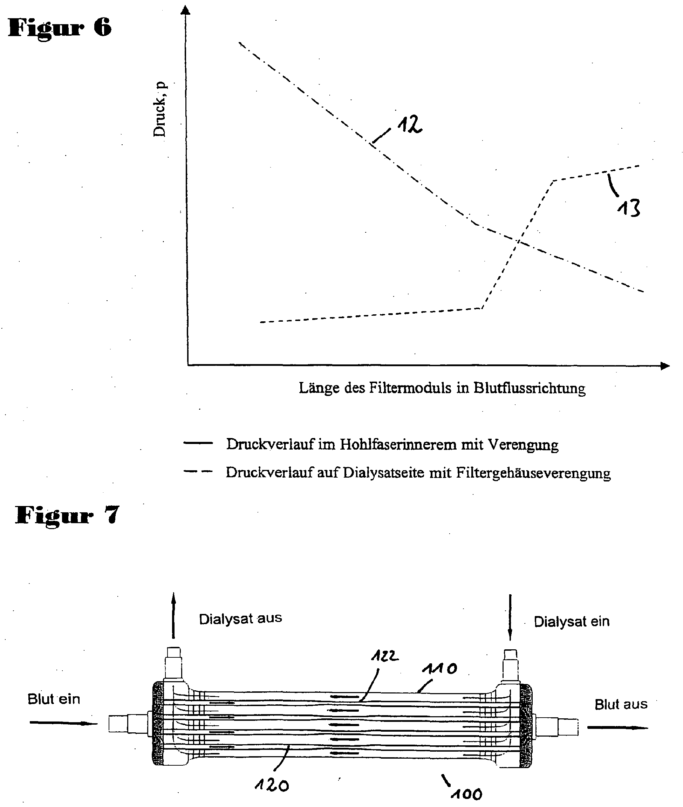

Die Druckzustände, die in einem Filtermodul auf Blut- und Dialysatseite vorzufinden sind, bei dem des Weiteren auf der Dialysatseite eine Verengung des Filtergehäuses, d.h. eine bereichsweise Verringerung des Strömungsquerschnitts auf der Dialysatseite ergibt, sind in Figur 6 dargestellt, wobei die Linie 12 den Druckverlauf auf der Blutseite und Linie 13 den Druckverlauf auf der Dialysatseite, jeweils in Blutflußrichtung zeigen.

Wie sich dies bei einem Vergleich der Darstellungen der Figur 2 und Figur 4 und Figur 6 ergibt, liegen die Druckkurven für Dialysat und Blut bis zur Engstelle des Faserbündels weiter auseinander als für den Fall, dass weder die Hohlfasern noch das Filtergehäuse eine Engstelle aufweist. Ein besonders großer Abstand zwischen beiden Druckkurven ergibt sich für den Fall, dass die Hohlfasern eine Engstelle aufweisen als auch dass eine Filtergehäuseverengung oder eine sonstige Verringerung des Strömungsquerschnittes auf der Dialysatseite vorliegt, wie dies aus Figur 6 hervorgeht.

Figur 7 zeigt einen erfindungsgemäßen Dialysator (Filter) 100 mit einem Gehäuse 110 und darin parallel zur Längsrichtung des Gehäuses angeordneten Hohlfasern 120, die zu einem Bündel zusammengefaßt sind. Die Hohlfasern 120 befinden sich in ihren Endbereichen in Vergußmassen, die dichtend an der Innenseite des Gehäuses 110 anliegen. Wie dies weiter aus Figur 7 hervorgeht, werden die Faserinnenräume von Blut gemäß Figur 7 von links nach rechts und der Dialysatraum, der die Fasern 120 umgibt, von rechts nach links, d.h. im Gegenstrom von Dialysierflüssigkeit durchströmt. Bezogen auf die gesamte Länge der Hohlfasern weisen diese in etwa mittig eine Engstelle 122 auf, die sich über einen bestimmten Längenabschnitt der Hohlfasern 120 erstreckt. Der sich bei Einsatz eines Filters 100 gemäß Figur 7 ergebende Druckverlauf in durch die Linien 10 und 11 in Figur 4 wiedergegeben.

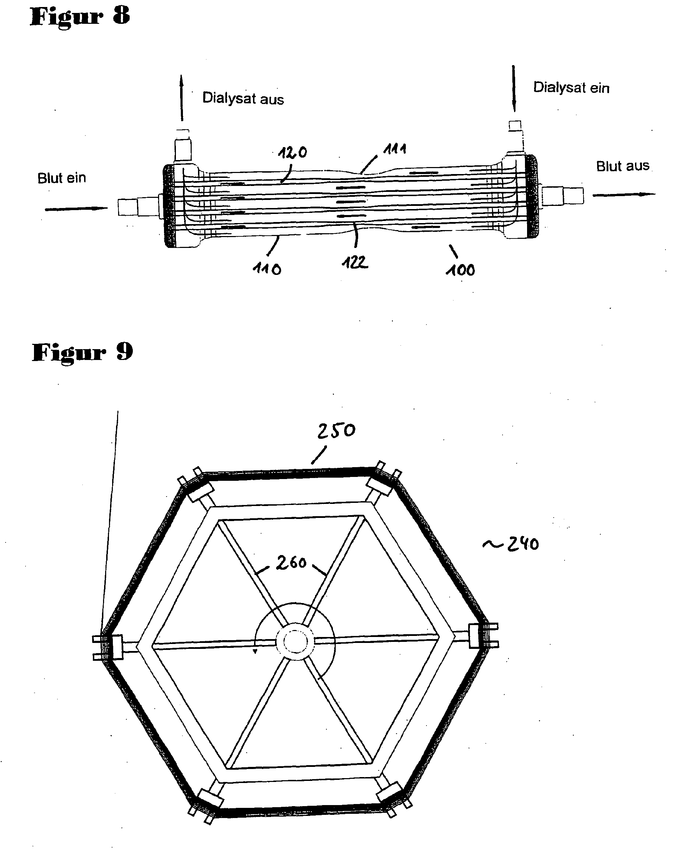

Figur 8 zeigt einen erfindungsgemäßen Dialysator (Filter) 100, der sich von dem Dialysator gemäß Figur 7 dadurch unterscheidet, dass das Gehäuse 110 eine Verjüngung 111 aufweist, in der der Gehäuseinnendurchmesser gegenüber den weiteren Abschnitten des Gehäuses verringert ist. Insgesamt ergibt sich durch diese Anordnung ein besonders hoher transmembraner Druck. Die Flußrestriktion auf der Dialysatseite ist in diesem Fall am Beispiel einer Einschnürung des Filtergehäuses dargestellt. Diese Darstellung gibt ein solches Filtermodul nur schematisch wieder. Wie oben ausgeführt, sind auch weitere Flussrestriktionsvorrichtungen auf der Dialysatseite vorstellbar. Diese können z. B. ein Ring sein, der das Faserbündel umschließt und im inneren eines zylindrischen Filtergehäuses liegt. Es kann sich aber auch um eine quellbare Substanz handeln, in die Fasern eingebettet sind und die im Kontakt mit dem Dialysat aufquellen und durch Flußrestriktion einen Drucksprung auf der Dialysatseite verursachen. Durch diese Maßnahme wird auf den Druckverlauf auf der Dialysatseite Einfluß genommen, wie sich dies aus einem Vergleich der Linien 4 und 5 in Figur 2 ergibt. Der Druckverlauf der Anordnung gemäß Figur 8 ist in Figur 6 wiedergegeben.

Die Lage der Engstelle im Faserbündel und der Flußrestriktion auf der Dialysatseite ist nicht auf die in Figur 8 gezeigte Ausführung beschränkt. Beide Engstellen können sich auch im dritten Drittel des Filtermoduls, betrachtet in Blutflußrichtung, befinden. Dadurch wird der TMP vor der Faserbündelengstelle weiter vergrößert und der konvektive Stofftransport von der Blut- auf die Dialysatseite erhöht. Ebenso können aber Faserengstelle und Dialysatflussrestriktion im ersten Drittel des Filtermoduls liegen. Bei einer solchen Anordnung wird der konvektive Stofftransport von der Dialysat- auf die Blutseite gefördert. Weitere relative Lagen der Faserengstelle zu Dialysatflussrestriktion sind möglich.

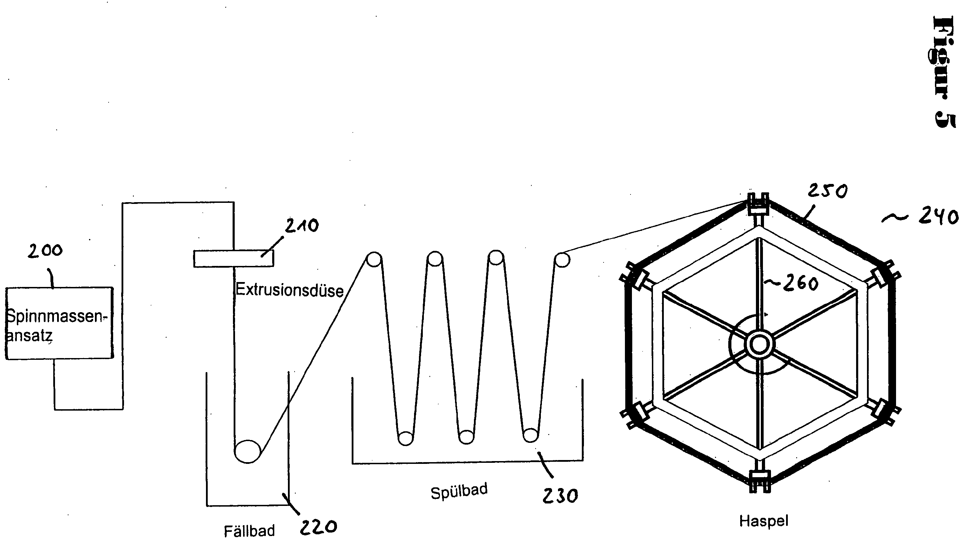

Figur 5 zeigt eine Anordnung zur Herstellung einer Hohlfaser bzw. eines Hohlfaserbündels gemäß der Erfindung. Mit dem Bezugszeichen 200 ist der Spinnmassenansatz gekennzeichnet. Mittels der Extrusionsdüse 210 wird ein Polymerlösungsfaden erzeugt. Es handelt sich bei der Düse 210 um eine Ringdüse, die in einem durch diese begrenzten Bereich eine weitere Düse aufweist, mittels derer Fällmittel ins Innere des Polymerlösungsfadens eingebracht wird. Beispielsweise durch Veränderung des Fällmitteldruckes, durch Veränderung der Abzugsgeschwindigkeit, mit der der Faden aus der Düse 210 abgezogen wird, oder durch hier nicht dargestellte Walzen kann der Innendurchmesser des Polymerlösungsfadens bzw. der Hohlfaser derart verändert werden, dass bezogen auf die fertige Hohlfaser eine Engstelle vorliegt, in der der Innendurchmesser der Hohlfaser verringert ist.

Die Hohlfaser mit Engstelle wird durch das Rollensystem der Spinnanlage durch das Fällbad 220 und das Wasch- oder Spülbad 230 transportiert.

Erfindungsgemäß ist vorgesehen, dass die Hohlfasermembran derart auf die Haspel 240 aufgewickelt wird, dass die Engstelle an der gewünschten Stelle, z.B. mittig oder zur Mitte versetzt, in einem Haspelsegment 250 abgelegt wird. Um dies zu gewährleisten, wird beim Durchlaufen eines Haspelarmes 260 ein Signal eines Näherungsschalters erzeugt. Da die Laufzeit (Totzeit) der Engstelle von der Extrusionsdüse oder Prägewalze bis zur Mitte bzw. zur gewünschten Position des Haspelsegments 250 bekannt ist, läßt sich die Rotationsgeschwindigkeit der Haspel 240 derart einstellen, dass die Engstelle an der gewünschten Position im Haspelsegment zu liegen kommt und sich damit auch an der gewünschten Position im Faserbündel befindet. Auch kann bei konstanter Rotationsgeschwindigkeit der Haspel die Fördergeschwindigkeit des Fadens so eingestellt werden, dass sich die Engstelle an der gewünschten Position befindet.

Weitere Optimierungen, die sich dem Fachmann aus dem gezeigten Lösungsansatz von selbst ergeben, sind zu beachten. So nimmt z. B. der effektive Haspelumfang durch die nach und nach abgelegten Hohlfasern zu. Dadurch ist es notwendig die Rotationsgeschwindigkeit der Haspel mit zunehmender Dauer des Spinnprozesses zu verlangsamen. Ebenso muss die Totzeit im Laufe des Spinnprozesses angepasst werden.

Das Faserbündel mit erfindungsgemäß angeordneter Engstelle in den Fasern kann dann aus einem Haspelsegment herausgeschnitten werden und steht der weiteren Verarbeitung zu einem Filtermodul zur Verfügung.

Figur 9 zeigt schließlich ein Haspelrad 240 mit Haspelarmen 260 und durch deren Enden begrenzte Haspelsegmente 250 in vergrößerter Darstellung.

Alle Abbildungen verstehen sich nur grob schematisch. Si sollen nur zeigen, dass sich gemäß den dargestellten Ausführungsformen vor einer Verengung im Strömungskanal ein Staudruck aufbaut und der Druckabfall dann in Flussrichtung langsamer erfolgt als ohne Verengung. An der Verengung erfolgt ein rapider Druckabfall. Nach der Verengung sollten die Strömungswiderstände, ob mit oder ohne Verengung, vorzugsweise gleich sein. Die Lage der Druckkurven für Hohlfaserinnenseite und Dialysatseite zueinander sind rein schematisch. Jedoch liegen Sie vorzugsweise zueinander so, dass wie in Figur 6 gezeigt durch die beiden Kurven eingeschlossene Flächen entstehen. Der Betrag der Fläche ist Maß für die Größe des konvektiven Transports durch die Membranwand. Die Blockpfeile gemäß Figur 6 geben an, in welche Richtung entsprechend der transmembranen Druckdifferenz der konvektive Transport erfolgt.

Die Steigungen in den einzelnen Abschnitten der Druckkurven sind ebenfalls nur schematisch zu verstehen.

## Patentansprüche

1. Dialysator (100) mit einem Filtergehäuse (110) und wenigstens einem in dem Filtergehäuse (110) angeordneten Faserbündel, wobei das Faserbündel eine Vielzahl von Hohlfasern (120) aus einem semipermeablen Membranmaterial aufweist, **dadurch gekennzeichnet, dass** die Hohlfasern (120) über ihre gesamte Länge jeweils genau eine Engstelle (122) aufweisen, in der der Innendurchmesser der Hohlfaser gegenüber den Abschnitten der Hohlfasern (120), die an die Engstelle (122) angrenzen, verringert ist, wobei die Engstellen (122) zwischen den Enden der Hohlfasern (120) angeordnet sind.

2. Dialysator (100) nach Anspruch 1, **dadurch gekennzeichnet, dass** sich die Engstellen (122) über einen Längenabschnitt der jeweiligen Hohlfaser erstrecken, wobei der Längenabschnitt weniger als 5 %, zwischen 5 % und 10 %, zwischen 10 % und 15 % oder zwischen 15 % und 25 % der gesamten Länge der Faser beträgt.

3. Dialysator (100) nach Anspruch 1 oder 2, **dadurch gekennzeichnet, dass** der Innendurchmesser der Hohlfasern (120) in den Engstellen (122) weniger als 40 %, zwischen 40 % und 50 % oder zwischen 50 % und 80 % des Innendurchmessers der jeweiligen Hohlfaser (120) in den nicht durch die Engstelle (112) gebildeten Abschnitten der Hohlfaser (120) beträgt.

4. Dialysator (100) nach einem der vorhergehenden Ansprüche, **dadurch gekennzeichnet**, sich die Engstellen (122) über einen Längenabschnitt der jeweiligen Hohlfaser (120) erstrecken und einen konstanten Innendurchmesser aufweisen.

5. Dialysator (100) nach einem der vorhergehenden Ansprüche, **dadurch gekennzeichnet**, sich die Engstellen (122) über einen Längenabschnitt der jeweiligen Hohlfaser (120) erstrecken und einen zunächst abnehmenden und dann wieder zunehmenden Innendurchmesser aufweisen.

6. Dialysator (100) nach einem der vorhergehenden Ansprüche, **dadurch gekennzeichnet, dass** der Übergang von den einer jeweiligen Engstelle benachbarten Bereichen zu der Engstelle (122) stufig oder stetig ist.

7. Dialysator (100) nach einem der vorhergehenden Ansprüche, **dadurch gekennzeichnet, dass** sich die Engstellen (122) über einen Längenabschnitt der jeweiligen Hohlfaser (120) erstrecken, dass ein oder zwei Übergangsbereiche zwischen der Engstelle (122) und nicht durch die Engstelle (122) gebildeten Bereichen der Hohlfaser (120) existieren und dass die Länge des der Übergangsbereiches zwischen 5 % und 20 % der Länge des genannten Längenabschnittes beträgt.

8. Dialysator (100) nach einem der vorhergehenden Ansprüche, **dadurch gekennzeichnet, dass** die Engstellen (122) eine Stelle minimalen Innendurchmessers der jeweiligen Hohlfaser (120) aufweisen und dass die Engstellen (122) zu dieser Stelle symmetrisch sind.

9. Dialysator (100) nach einem der vorhergehenden Ansprüche, **dadurch gekennzeichnet, dass** Flussrestriktionsmittel vorgesehen sind, durch die die Fläche des die Hohlfasern (120) des Faserbündels umgebenden Bereiches innerhalb des Filtergehäuses (110) verringert wird.

10. Dialysator (100) nach Anspruch 9, **dadurch gekennzeichnet, dass** die Flussrestriktionsmittel als Engstelle (122) des Filtergehäuses (110) ausgeführt sind, in der der Innendurchmesser des Filtergehäuses (110) gegenüber dem oder den Abschnitten des Gehäuses, die an die Engstelle (122) angrenzen, verringert ist.

11. Dialysator (100) nach Anspruch 9 oder 10, **dadurch gekennzeichnet, dass** die Flussrestriktionsmittel als Ring ausgeführt sind, der innerhalb des Filtergehäuses (110) angeordnet ist und der das Faserbündel umgibt.

12. Filter nach einem der Ansprüche 9 bis 11, **dadurch gekennzeichnet, dass** die Flussrestriktionsmittel als quellbare Substanz ausgeführt sind, die bei Kontakt mit einem Medium, das den das Faserbündel umgebenden Raum durchströmt, aufquellen.

13. Verfahren zur Herstellung eines Dialysators (100) nach einem der vorhergehenden Ansprüche, wobei das Verfahren das Aufwickeln der Hohlfaser (120) oder des Hohlfaserbündels auf eine Aufwickelvorrichtung, insbesondere auf eine Haspel (240) umfasst,
**dadurch gekennzeichnet, dass**
eine Engstelle (122) der Hohlfaser (120) erzeugt wird, in der der Innendurchmesser der Hohlfaser (120) gegenüber dem oder den Abschnitten der Hohlfaser (120), die an die Engstelle (122) angrenzen, verringert ist und dass die Stellung der Aufwickelvorrichtung derart mit der Erzeugung der Engstelle (122) der Hohlfaser (120) synchronisiert wird, dass sich die Engstelle (122) an einer vorgegebenen Position relativ zu der Aufwickelvorrichtung befindet, bei Erreichen wenigstens einer vorgegebenen Position der Aufwickelvorrichtung, insbesondere eines Haspelarmes (260) einer Haspel (240), ein Signal erzeugt wird und dass auf der Grundlage des Signals die Erzeugung der Engstelle (122) vorgenommen wird, und
die Engstelle (122) der Hohlfaser (120) dadurch erzeugt wird, dass der Fällmitteldruck des im Inneren des Polymerlösungsfadens, aus dem die Hohlfaser (120) gebildet wird, befindlichen Fällmittels vorübergehend verringert wird, das die Abzugsgeschwindigkeit, mit der der Polymerlösungsfaden, aus dem die Hohlfaser (120) gebildet wird, abgezogen wird, vorübergehend erhöht wird, oder dass von außen Präge- und/oder Riffelwalzen auf den Polymerlösungsfaden oder die daraus gebildete Hohlfaser (120) einwirken.

14. Verfahren nach Anspruch 13, **dadurch gekennzeichnet, dass** es sich bei der Aufwickelvorrichtung um eine Haspel (240) handelt, die wenigstens ein Haspelsegment (250) aufweist, und dass die Synchronisation der Erzeugung der Engstelle (122) mit der Position der Haspel (240) derart vorgenommen wird, dass die Engstellen (122) jeweils mittig in den Haspelsegmenten (250) liegen.

15. Verfahren nach Anspruch 13, **dadurch gekennzeichnet, dass** es sich bei der Aufwickelvorrichtung um eine Haspel (240) handelt, die wenigstens ein Haspelsegment (250) aufweist, und dass die Synchronisation der Erzeugung der Engstelle (122) mit der Position der Haspel (240) derart vorgenommen wird, dass die Engstellen (122) jeweils nicht mittig in den Haspelsegmenten (250) liegen.

16. Verfahren nach Anspruch 15, **dadurch gekennzeichnet, dass** die Synchronisation der Erzeugung der Engstelle (122) mit der Position der Haspel (240) derart vorgenommen wird, dass die Engestellen jeweils im ersten, zweiten oder dritten Drittel der Gesamtlänge der hergestellten Hohlfasern (120) liegen.

17. Verfahren nach Anspruch 13, **dadurch gekennzeichnet, dass** es sich bei der Aufwickelvorrichtung um eine Haspel (240) handelt, die wenigstens ein Haspelsegment (250) aufweist, und dass die Synchronisation der Erzeugung der Engstelle (122) mit der Position der Haspel (240) derart vorgenommen wird, dass pro Haspelsegment (250) mehr als eine Engstelle (122) der Hohlfasern (120) vorliegt.

## Claims

1. A dialyzer (100) comprising a filter housing (110) and at least one fiber bundle arranged in the filter housing (110), wherein the fiber bundle has a plurality of hollow fibers (120) made of a semipermeable membrane material,

**characterized in that**
the hollow fibers (120) have exactly one restriction (122) over their total length in which the inner diameter of the hollow fiber is reduced with respect to the sections of the hollow fibers (120) adjoining the restriction (122), with the restrictions (122) being arranged between the ends of the hollow fibers (120).

2. A dialyzer (100) in accordance with claim 1, wherein the restrictions (122) extend over a longitudinal section of the respective hollow fiber, with the longitudinal section amounting to less than 5%, between 5% and 10%, between 10% and 15% or between 15% and 25% of the total length of the fiber.

3. A dialyzer (100) in accordance with either of claims 1 or 2, wherein the inner diameter of the hollow fibers (120) in the restrictions (122) amounts to less than 40%, between 40% and 50% or between 50% and 80% of the inner diameter of the respective hollow fiber (120) in the sections of the hollow fiber (120) not formed by the restriction (112).

4. A dialyzer (100) in accordance with one of the preceding claims, wherein the restrictions (122) extend over a longitudinal section of the respective hollow fiber (120) and have a constant inner diameter.

5. A dialyzer (100) in accordance with one of the preceding claims, wherein the restrictions (122) extend over a longitudinal section of the respective hollow fiber (120) and have a first decreasing and then again increasing inner diameter.

6. A dialyzer (100) in accordance with one of the preceding claims, wherein the transition from the regions adjacent to the respective restriction to the restriction (122) is stepped or constant.

7. A dialyzer (100) in accordance with one of the preceding claims, wherein the restrictions (122) extend over a longitudinal section of the respective hollow fiber (120); wherein one or two transitional regions exist between the restriction (122) and regions of the hollow fiber (120) not formed by the restriction (122); and wherein the length of the transitional region amounts to between 5% and 20% of the length of the said longitudinal section.

8. A dialyzer (100) in accordance with one of the preceding claims, wherein the restrictions (122) have a point of minimal inner diameter of the respective hollow fiber (120); and wherein the restrictions (122) are symmetrical to this point.

9. A dialyzer (100) in accordance with one of the preceding claims, wherein flow restriction means are provided by which the surface of the region surrounding the hollow fibers (120) of the fiber bundle is reduced within the filter housing (110).

10. A dialyzer (100) in accordance with claim 9, wherein the flow restriction means are configured as a restriction (122) of the filter housing (110) in which the inner diameter of the filter housing (110) is reduced with respect to the section or sections of the housing adjoining the restriction (122).

11. A dialyzer (100) in accordance with either of the claims 9 or 10, wherein the flow restriction means are configured as a ring which is arranged within the filter housing (110) and which surrounds the fiber bundle.

12. A filter in accordance with one of the claims 9 to 11, wherein the flow restriction means are configured as a swellable substance which swell up on contact with a medium which flows through the space surrounding the fiber bundle.

13. A method for the manufacture of a dialyzer (100) in accordance with one of the preceding claims, wherein the method comprises the winding up of the hollow fiber (120) or of the hollow fiber bundle onto a wind-up device, in particular onto a reel (240),
**characterized in that**
a restriction (122) of the hollow fiber (120) is produced in which the inner diameter of the hollow fiber (120) is reduced with respect to the section or sections of the hollow fiber (120) adjoining the restriction (122); and **in that** the position of the wind-up device is synchronized with the production of the restriction (122) of the hollow fiber (120) such that the restriction (122) is located at a predetermined position relative to the wind-up device;
on the reaching of at least one predetermined position of the wind-up device, in particular of a reel arm (260) of a reel (240), a signal is produced; and **in that** the production of the restriction (122) is carried out on the basis of the signal; and
the restriction (122) of the hollow fiber (120) is produced **in that** the precipitating agent pressure of the precipitating agent located in the interior of the polymer solution thread from which the hollow fiber (120) is formed is temporarily reduced; **in that** the removal speed at which the polymer solution thread from which the hollow fiber (120) is formed is removed is temporarily increased; or **in that** stamping rolls and/or fluted rolls act from outside onto the polymer solution thread or the hollow fiber (120) formed therefrom.

14. A method in accordance with claim 13, wherein the wind-up device is a reel (240) which has at least one reel segment (250); and wherein the synchronization of the production of the restriction (122) with the position of the reel (240) is carried out such that the restrictions (122) each lie centrally in the reel segments (250).

**15.** A method in accordance with claim 13, wherein the wind-up device is a reel (240) which has at least one reel segment (250); and wherein the synchronization of the production of the restriction (122) with the position of the reel (240) is carried out such that the restrictions (122) each do not lie centrally in the reel segments (250).

**16.** A method in accordance with claim 15, wherein the synchronization of the production of the restriction (122) with the position of the reel (240) is carried out such that restrictions each lie in the first, second or third third of the total length of the manufactured hollow fibers (120).

**17.** A method in accordance with claim 13, wherein the wind-up device is a reel (240) which has at least one reel segment (250); and wherein the synchronization of the production of the restriction (122) with the position of the reel (240) is carried out such that more than one restriction (122) of the hollow fibers (120) is present per reel segment (250).

**Revendications**

**1.** Dialyseur (100) comprenant un boîtier de filtre (110) et au moins un faisceau de fibres disposé dans le boîtier de filtre (110), le faisceau de fibres comportant une pluralité de fibres creuses (120) constituées d'un matériau de membrane semi-perméable, **caractérisé en ce que**
les fibres creuses (120) comportent sur toute leur longueur respectivement exactement un point de resserrement (122), dans lequel le diamètre intérieur de la fibre creuse est diminué par rapport aux portions des fibres creuses (120), qui sont adjacentes au point de resserrement (122), les points de resserrement (122) étant disposés entre les extrémités des fibres creuses (120).

**2.** Dialyseur (100) selon la revendication 1, **caractérisé en ce que** les points de rétrécissement (122) s'étendent sur une portion de longueur de la fibre creuse respective, la portion de longueur correspondant à moins de 5 %, entre 5 % et 10 %, entre 10 % et 15 % ou entre 15 % et 25 % de la longueur totale de la fibre.

**3.** Dialyseur (100) selon la revendication 1 ou 2, **caractérisé en ce que** le diamètre intérieur des fibres creuses (120) dans les points de resserrement (122) correspond à moins de 40 %, entre 40 % et 50 % ou entre 50 % et 80 % du diamètre intérieur de la fibre creuse (120) respective dans les portions de la fibre creuse (120) qui ne sont pas formées par le point de resserrement (112).

**4.** Dialyseur (100) selon l'une des revendications précédentes, **caractérisé en ce que** les points de resserrement (122) s'étendent sur une portion de longueur de la fibre creuse (120) respective et présentent un diamètre intérieur constant.

**5.** Dialyseur (100) selon l'une des revendications précédentes, **caractérisé en ce que** les points de resserrement (122) s'étendent sur une portion de longueur de la fibre creuse (120) respective et présentent un diamètre intérieur qui est d'abord décroissant puis de nouveau croissant.

**6.** Dialyseur (100) selon l'une des revendications précédentes, **caractérisé en ce que** la transition des zones voisines d'un point de resserrement respectif au point de resserrement (122) est discontinue ou continue.

**7.** Dialyseur (100) selon l'une des revendications précédentes, **caractérisé en ce que** les points de resserrement (122) s'étendent sur une portion de longueur de la fibre creuse (120) respective, **en ce qu'**une ou deux zones de transition entre le point de resserrement (122) et des zones de la fibre creuse (120) qui ne sont pas formées par le point de resserrement (122) existent et **en ce que** la longueur de la zone de transition correspond à entre 5 % et 20 % de la longueur de ladite portion de longueur.

**8.** Dialyseur (100) selon l'une des revendications précédentes, **caractérisé en ce que** les points de resserrement (122) comportent un point avec un diamètre intérieur minimal de la fibre creuse (120) respective et **en ce que** les points de resserrement (122) sont symétriques par rapport à ce point.

**9.** Dialyseur (100) selon l'une des revendications précédentes, **caractérisé en ce que** des moyens de restriction de flux sont prévus, par lesquels la surface de la zone entourant les fibres creuses (120) du faisceau de fibres est diminuée à l'intérieur du boîtier de filtre (110).

**10.** Dialyseur (100) selon la revendication 9, **caractérisé en ce que** les moyens de restriction de flux sont réalisés sous la forme d'un point de resserrement (122) du boîtier de filtre (110), dans lequel le diamètre intérieur du boîtier de filtre (110) est diminué par rapport à la ou aux portion(s) du boîtier, qui est/sont adjacente(s) au point de resserrement (122).

**11.** Dialyseur (100) selon la revendication 9 ou 10, **caractérisé en ce que** les moyens de restriction de flux sont réalisés sous la forme d'un anneau, qui est disposé à l'intérieur du boîtier de filtre (110) et qui entoure le faisceau de fibres.

12. Filtre selon l'une des revendications 9 à 11, **caractérisé en ce que** les moyens de restriction de flux sont réalisés sous la forme d'une substance apte au gonflement, qui gonfle en cas de contact avec un milieu qui traverse l'espace entourant le faisceau de fibres.

13. Méthode de production d'un dialyseur (100) selon l'une des revendications précédentes, dans laquelle la méthode comprend l'enroulement des fibres creuses (120) ou du faisceau de fibres creuses sur un dispositif d'enroulement, en particulier sur un dévidoir (240),
   **caractérisée**
   **en ce qu'**un point de resserrement (122) de la fibre creuse (120) est généré, dans lequel le diamètre intérieur de la fibre creuse (120) est diminué par rapport à la ou aux portion(s) de la fibre creuse (120), qui est/sont adjacente(s) au point de resserrement (122) et **en ce que** la position du dispositif d'enroulement est synchronisée avec la génération du point de resserrement (122) de la fibre creuse (120) de telle manière que le point de resserrement (122) se trouve dans une position prédéfinie par rapport au dispositif d'enroulement,
   **en ce qu'**un signal est généré lorsqu'au moins une position prédéfinie du dispositif d'enroulement, en particulier d'un bras de dévidoir (260) d'un dévidoir (240), est atteinte et **en ce que** la génération du point de resserrement (122) est effectuée sur la base du signal, et
   **en ce que** le point de resserrement (122) de la fibre creuse (120) est généré par le fait que la pression du précipitant se trouvant à l'intérieur du fil de solution de polymère, à partir duquel la fibre creuse (120) est formée, est temporairement diminuée, que la vitesse de tirage, avec laquelle le fil de solution de polymère, à partir duquel la fibre creuse (120) est formée, est tiré, est temporairement augmentée, ou que des rouleaux de gaufrage et/ou des rouleaux cannelés agissent de l'extérieur sur le fil de solution de polymère ou la fibre creuse (120) formée à partir de celui-ci.

14. Méthode selon la revendication 13, **caractérisée en ce que** le dispositif d'enroulement est un dévidoir (240), qui comporte au moins un segment de dévidoir (250), et **en ce que** la synchronisation de la génération du point de resserrement (122) avec la position du dévidoir (240) est effectuée de telle manière que les points de resserrement (122) se trouvent respectivement au milieu des segments de dévidoir (250).

15. Méthode selon la revendication 13, **caractérisée en ce que** le dispositif d'enroulement est un dévidoir (240), qui comporte au moins un segment de dévidoir (250), et **en ce que** la synchronisation de la génération du point de resserrement (122) avec la position du dévidoir (240) est effectuée de telle manière que les points de resserrement (122) ne se trouvent respectivement pas au milieu des segments de dévidoir (250).

16. Méthode selon la revendication 15, **caractérisée en ce que** la synchronisation de la génération du point de resserrement (122) avec la position du dévidoir (240) est effectuée de telle manière que les points de resserrement se trouvent respectivement dans le premier, le deuxième ou le troisième tiers de la longueur totale des fibres creuses (120) fabriquées.

17. Méthode selon la revendication 13, **caractérisée en ce que** le dispositif d'enroulement est un dévidoir (240), qui comporte au moins un segment de dévidoir (250), et **en ce que** la synchronisation de la génération du point de resserrement (122) avec la position du dévidoir (240) est effectuée de telle manière qu'il y a plus d'un point de resserrement (122) des fibres creuses (120) par segment de dévidoir (250).

# Figur 1

Druck, p

Länge des Filtermoduls in Blutflussrichtung, L

—— Druckverlauf im Hohlfaserinnerem

— — Druckverlauf auf Dialysatseite

# Figur 2

Länge des Filtermoduls in Blutflussrichtung

——— Druckverlauf im Hohlfaserinnerem

— — Druckverlauf auf Dialysatseite Dialysat

- - - Druckverlauf auf Dialysatseite mit Filtergehäuseverengung

# Figur 3

Länge des Filtermoduls in Blutflussrichtung

——— Druckverlauf im Hohlfaserinnerem mit Radius $r_1$

— — Druckverlauf auf Dialysatseite

— · — Druckverlauf im Hohlfaserinnerem mit verringertem Radius $r_2$; $r_2 < r_1$

# Figur 4

Länge des Filtermoduls in Blutflussrichtung

—— Druckverlauf im Hohlfaserinnerem mit gleich bleibendem Radius

— — Druckverlauf auf Dialysatseite

— · — Druckverlauf im Hohlfaserinnerem mit Verjüngung

# Figur 5

## Figur 6

Länge des Filtermoduls in Blutflussrichtung

—— Druckverlauf im Hohlfaserinnerem mit Verengung

— — Druckverlauf auf Dialysatseite mit Filtergehäuseverengung

## Figur 7

## Figur 8

Dialysat aus

Dialysat ein

Blut ein

Blut aus

120

111

110

122

100

## Figur 9

250

~240

260

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5730712 A
- EP 1433490 A
- EP 1344542 A
- WO 2006024902 A
- WO 03106004 A
- JP H0796152 A

- EP 0750936 B1
- EP 1547628 A1
- EP 0543355 B1
- DE 2842958 A1
- US 4380520 A
- US 4291096 A

**In der Beschreibung aufgeführte Nicht-Patentliteratur**

- **C. RONCO et al.** Enhancement of convective transport by internal filtration in a modified experimental hemodialyzer. *Kidney International,* 1998, vol. 54, 979

- **RONCO et al.** Effects of a reduced inner diameter of hollow fibers in hemodialyzers. *Kidney International,* 2000, vol. 58, 809